(19) Europäisches Patentamt
European Patent Office
Office européen des brevets

(11) Veröffentlichungsnummer : **0 537 430 B1**

# (12) EUROPÄISCHE PATENTSCHRIFT

(45) Veröffentlichungstag der Patentschrift : **08.06.94 Patentblatt 94/23**

(51) Int. Cl.$^5$ : **A61K 35/10**

(21) Anmeldenummer : **92113140.5**

(22) Anmeldetag : **01.08.92**

## (54) Antivirale Mittel.

(30) Priorität : **17.10.91 DE 4134378**

(43) Veröffentlichungstag der Anmeldung : **21.04.93 Patentblatt 93/16**

(45) Bekanntmachung des Hinweises auf die Patenterteilung : **08.06.94 Patentblatt 94/23**

(84) Benannte Vertragsstaaten : **AT BE CH DE DK ES FR GB GR IT LI LU NL PT SE**

(56) Entgegenhaltungen :
**EP-A- 0 281 678**
**EP-A- 0 281 679**
**EP-A- 0 313 718**
**Z. ALLG. MIKROBIOL. Bd. 15, Nr. 1, 1975, Seiten 25 - 30; RENATE KLÖCKING ET AL.: 'Wirkung von Ammoniumhumat auf einige Virus-Zell-Systeme'**

(56) Entgegenhaltungen :
**PHARMAZIE Bd. 34, Nr. 5-6, 1979, Seiten 292 - 293; RENATE KLÖCKING ET AL.:'Gewinnung, Charakterisierung und antivirale Aktivität von-Phenolkörperpolymerisaten'**
**Biosis Abstract Nr. 43102365 Schneider J. et al: Inhibition of HIV-1 infectionby synthetic huminates**

(73) Patentinhaber : **RÜTGERSWERKE AKTIENGESELLSCHAFT**
**Mainzer Landstrasse 217**
**D-60326 Frankfurt (DE)**

(72) Erfinder : **Riede,Urs N. Prof. Dr.**
**Bifänge 89**
**D-7800 Freiburg (DE)**
Erfinder : **Seubert, Bernd**
**Meisenweg, 15**
**D-6803 Edingen (DE)**
Erfinder : **Schneider, Josef**
**Schneeburgstrasse, 62**
**D-7800 Freiburg (DE)**

EP 0 537 430 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue antivirale Mittel, insbesondere solche, die gegen Retroviren wirksam sind.

Virale Infektionen gelten als chemotherapeutisch schwierig beherrschbar. Dies ist daraufzurückzuführen, daß Viren überwiegend den anabolischen Stoffwechsel der befallenen Zellen zu ihrer eigenen Vermehrung ausnutzen. Viele Substanzen, die antiviral wirksam sind, hemmen deswegen gleichzeitig auch den zellulären Stoffwechsel und sind toxisch.

Als Angriffspunkte für eine antivirale Chemotherapie eignen sich eine Vielzahl Virus-spezifischer Reaktionsabläufe wie z. B.
- die Bindung des Virus an seinen Rezeptor, - die Vermehrung der viralen Nukleinsäuren, - die Regulation des viralen Genexpression und - die Morphogenese des Viruspartikels. In-vitro ließen sich durch Inhibition dieser Vorgänge die verschiedensten Viren blockieren.

In-vivo haben sich bisher jedoch nur wenige antivirale Wirkstoffe bewährt. Die Gründe dafür liegen in den hohen Anforderungen sowohl an Spezifität als auch an die Wirkungsbreite antiviraler Wirkstoffe. Nur bei hoher Spezifität werden die viralen Reaktionsketten blockiert, ohne die mit ihnen häufig verwandten Reaktionsabläufe der Zelle zu schädigen. Die hohe Spezifität bedingt aber meist auch eine hohe Selektivität dem Virus gegenüber, das heißt, die Wirkung richtet sich nur gegen eine Virusspezies oder sogar gegen ein Isolat dieser Spezies. Viren können also unter dem Selektionsdurck der Substanz leicht resistent werden, oder die Viruspopulationen eines Patienten enthalten bereits resistente Varianten, die sich bei Anwendung der Wirkstoffe selektiv vermehren.

Aus Z. allg. Mikrobiol. 15 (1975), 25 bis 30 ist bekannt, daß Ammoniumhuminate aus natürlichen Huminstoffen eine antivirale Wirkung mit einem breiten Spektrum gegen Viren verschiedenster Art darstellen und andererseits eine geringe zellschädigende Wirkung zeigen.

Desgleichen ist aus Pharmazie 34 (1979), 292 bis 293 bekannt, daß synthetische Huminstoffe, die durch Oxiation mehrwertiger Phenole hergestellt wurden, ähnliche antivirale Eigenschaften besitzen.

Obwohl die Verträglichkeit dieser Stoffgruppe sehr gut ist, ist die viruzide Wirksamkeit noch nicht zufriedenstellend.

Es ist deshalb Aufgabe der Erfindung, antivirale Mittel bereitzustellen, die den gesunden tierischen oder menschlichen Stoffwechsel möglichst wenig schädigen und dabei gegen Viren verschiedenster Art stärker wirksam sind als natürliche Huminate oder solche, die durch Oxidation mehrwertiger Phenole hergestellt wurden.

Die Lösung der Aufgabe besteht in der Bereitstellung und Verwendung neuer synthetischer, modifizierter Huminate gemäß der Ansprüche 1 bis 3.

Diese modifizierten Huminate werden hergestellt durch Oxidation der Verbindungen der allgemeinen Formeln

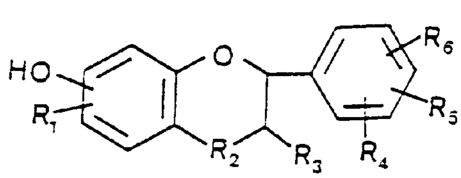

I

und/oder

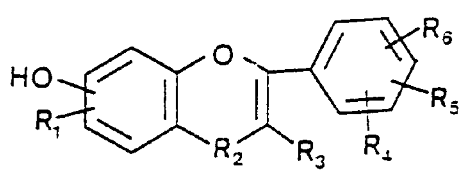

II

wobei $R_1$, $R_3$ und $R_4$ gleich oder verschieden sind und eine OH-Gruppe oder Wasserstoff darstellen, $R_2$ eine CO- oder $CH_2$-Gruppe bedeutet und $R_5$ und $R_6$ gleich oder verschieden sind und Wasserstoff, eine OH- oder Methoxygruppe darstellen, wobei $R_4$, $R_5$ und $R_5$ nicht alle gleichzeitig Wasserstoff sind, in alkalischer, wäßriger Lösung (pH>9).

Sie sind physiologisch positiv wirksam, das heißt, sie haben heilende Eigenschaften, sind sehr gering toxisch und weder mutagen noch teratogen (Ames-Test).

Es wurde gefunden, daß sie sowohl bei Menschen als auch bei Tieren eine verstärkte antivirale Wirkung zeigen.

Beispiele hierfür sind antivirale Effekte

gegen Bienen-Paralyseviren,

gegen Herpesviren beim Menschen und (Herpes labialis) und Karpfen (sog. Karpfenpocken)

gegen HIV-Viren und andere Retroviren.

In-vitro Versuche haben gezeigt, daß erfindungsgemäße Huminate die Infektiosität der HIV-1 Viren unterdrücken ($IC_{50}$ = 0,05 µg/ml). Als Testsystem diente der Syncytien-essay mit MT-2-Zellen und MOLT-4(8)-Zellen. Da sowohl der Eintritt der Viruspartikel in die Zielzelle, als auch die Syncytienbildung in Form einer Membranfusion ablaufen, läßt sich vermuten, daß Huminate auf membranständige Strukturen einwirken. Daneben besitzen Huminate offenbar noch einen weiteren Angriffspunkt: Sie hemmen die reverse Transkriptase der HIV-1-Viren. Diesen Befunden zufolge ist es möglich, mit Huminaten einen oder mehrere Schritte des HIV-Infektionszyklus, sowie einen Schritt der HIV-induzierten Zytopathogenität zu blockieren.

Überraschenderweise wurde gefunden, daß modifizierte Ammonium- oder Alkalihuminate gegenüber den natürlichen und den durch Oxidation mehrwertigen Phenole hergestellten Huminaten eine bis um den Faktor 10 gesteigerte antivirale Wirksamkeit zeigen. Diese modifizierten Huminate, die Gegenstand einer parallelen Anmeldung der Anmelderin sind, werden hergestellt durch Oxidation der Verbindungen der allgemeinen Formeln

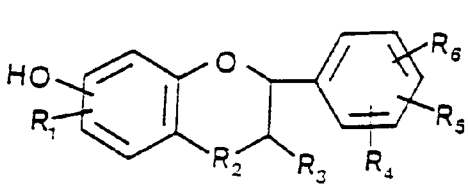

und/oder

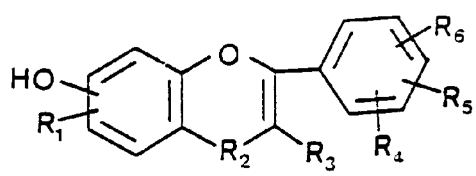

I

II

wobei $R_1$, $R_3$ und $R_4$ gleich oder verschieden sind und eine OH-Gruppe oder Wasserstoff darstellen, $R_2$ eine CO- oder $CH_2$-Gruppe bedeutet und $R_5$ und $R_6$ gleich oder verschieden sind und Wasserstoff, eine OH- oder Methoxygruppe darstellen, wobei $R_4$, $R_5$ und $R_5$ nicht alle gleichzeitig Wasserstoff sind, in alkalischer, wäßriger Lösung (pH > 9).

Dabei können die Verbindungen gemäß der Formeln I und II als einzelne Reinsubstanzen sowie auch im beliebigen Gemisch miteinander eingesetzt werden. Sie können im Reaktionsgemisch auch mit mehrwertigen Phenolen eingesetzt werden, was aus wirtschaftlichen Gründen durchaus angebracht sein kann.

## BEISPIELE

### Beispiel 1 (Vergleichsbeispiel)

In einem gegen Alkalien beständigen Rührwerkbehälter werden 95 kg Kaliumhydroxid (DAB 9-Qualität) in 1000 l entmineralisiertem Wasser (pH 5 bis 7; Leitfähigkeit unter 5 µS/cm) gelöst.

Nach dem Abkühlen auf eine Temperatur unterhalb 40 °C werden unter Kühlen im Verlauf von 45 Minuten 55 kg Hydrochinon (chem. rein., über 99 %) zugegeben, wobei die Temperatur des Gemisches 40 °C nicht übersteigt. Es stellt sich ein pH-Wert von etwa 10 ein.

Die Apparatur wird nun verschlossen. Dann wird unter Rühren Luft, die über ein alkalisches Filtersystem von Staub, $CO_2$ und anderen Kontaminanten gereinigt wird, über die Oberfläche geleitet.

Der Luftstrom wird so gedrosselt, daß der Gehalt an 1,4-Benzochinon immer kleiner ist als 0,5 %, bezogen auf das eingesetzte Hydrochinon. Die Reaktionstemperatur wird immer unter 40 °C gehalten. Der pH-Wert bleibt immer über 9.

Nach 10 bis 15 Tagen sinkt der Gehalt an Hydrochinon der braunen Reaktionslösung auf unter 2 %. Trotz weiterer Luftzufuhr sinkt der Gehalt an 1,4-Benzochinon auf Werte unter 0,05 %. In diesem Zeitpunkt wird die Reaktion abgebrochen. Die Luftzufuhr wird beendet und die Reaktionsmischung wird durch Zugabe eines sauren Ionenaustausches auf einen pH-Wert von 4,5 eingestellt. Danach wird der Ionenaustauscher durch zentrifugieren abgetrennt und die saure Lösung einer Ultrafiltration unterworfen, wobei ein Filtrationsgerät mit 0,5 µm Feinheit verwendet wird.

Das Ultrafiltrat wird unter schwachem Vakuum bis zu einer Huminat-Konzentration von 40 Gew.-% eingeengt. Diese Lösung ist noch gut handhabbar. Sie enthält Huminate mit Molekulargewichten im Bereich 1000 bis 50 000. Die Lösung zeigt keinen Tyndall-Effekt und enthält keine fluoreszierenden Komponenten.

Beispiel 2

29 g (0,1 Mol) Catechin (2-3,4-Dihydroxyphenyl)-chroman-3,5,7-triol) werden bei Raumtemperatur in 1000 ml einer wäßrigen 0,8 molaren Natronlaugelösung gelöst.

Die so erhaltene Lösung (pH 10) wird in einen mit Rührer, Thermometer, Gaseinführungs- und Überdruckentlastungs-Vorrichtung versehenen Glasreaktor gebracht. Danach wird bei Raumtemperatur durch die Lösung in ständigem Gasstrom Luft geleitet, die zuvor in einer Gaswaschflasche mit NaOH-Lösung von $CO_2$ und Staubpartikeln gereinigt wurde.

Der Gasstrom wird so reguliert, daß sich eine Temperatur der Reaktionslösung von 30 °C einstellt.

Nach einer Reaktionszeit von 10 Tagen, wenn trotz weiterer Luftzufuhr die Temperatur der Reaktionslösung absinkt, ist die Umsetzung beendet. Die entstandene dunkelbraune Lösung wird durch Zugabe von verdünnter Essigsäure auf eine pH-Wert von 6,0 eingestellt und danach mittels einer Ultrafiltration gereinigt.

Die gereinigte Lösung enthält 4,2 % eines modifizierten Huminats mit einem mittleren Molekulargewicht von 2 500 D bei einer Streubreite von 1 000 bis 30 000 D. Die Lösung ist dunkelbraun, fluoresziert nicht und zeigt keinen Tyndall-Effekt.

Beispiel 3

Huminate der Beispiele 1 und 2 werden auf ihre Wirkung hinsichtlich der Infektiosität von HIV-1 Viren getestet. Als Test-System dient der Syncytien-assay mit MT-2-Zellen und MOLT-4(8)-Zellen.

Erhalten werden folgende Ergebnisse:

| Huminat aus Beispiel | $IC_{50}$ [µg/ml] | $CC_{50}$ [µg/ml] |
|---|---|---|
| 1 | 0,15 | 200 |
| 2 | 0,05 | 25 |

**Patentansprüche**

1. Huminate enthaltende antivirale Mittel, **dadurch gekennzeichnet**, daß sie Huminate enthalten, die hergestellt sind durch Oxidation von Verbindungen der allgemeinen Formeln,

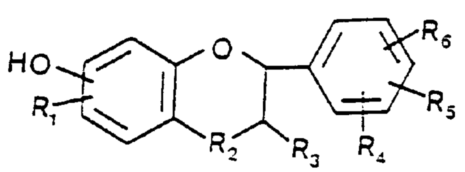

I

und/oder

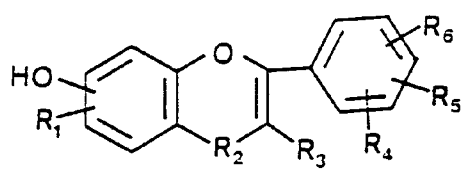

II

wobei $R_1$, $R_3$ und $R_4$ gleich oder verschieden sind und eine OH-Gruppe oder Wasserstoff darstellen, $R_2$ eine CO- oder $CH_2$-Gruppe bedeutet und $R_5$ und $R_5$ gleich oder verschieden sind und Wasserstoff, eine OH- oder Methoxygruppe darstellen, wobei $R_4$, $R_5$ und $R_5$ nicht alle gleichzeitig Wasserstoff sind, in alkalischer, wäßriger Lösung.

2. Verwendung der Huminate gemäß Anspruch 1 zur Herstellung eines Mittels gegen Retroviren.

3. Verwendung der Huminate gemäß Anspruch 1 zur Herstellung eines Anti-HIV-Mittels.

## Claims

**1.** Antiviral agents containing huminates, **characterised in that** they contain huminates which are manufactured by oxidation of compounds of the general formulae:

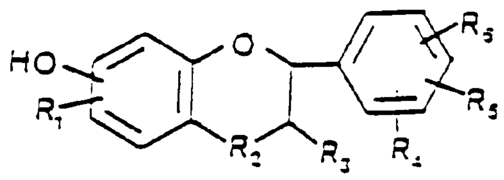 and/or 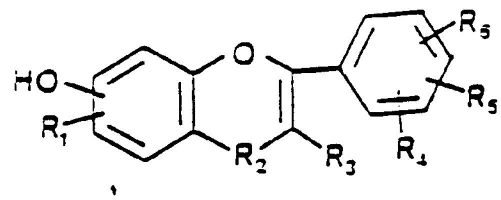

I

II

wherein $R_1$, $R_3$ and $R_4$ are the same or different and represent an OH-group or hydrogen, $R_2$ signifies a CO- or $CH_2$ group and $R_5$ and $R_5$ are the same or different and represent hydrogen, an OH-group or a methoxy group, $R_4$, $R_5$ and $R_6$ not all being hydrogen at the same time, in an alkaline aqueous solution.

**2.** Use of the huminates in accordance with claim 1 to produce an agent against retroviruses.

**3.** Use of the huminates in accordance with claim 1 to produce an anti- HIV agent.

## Revendications

**1.** Agents antiviraux contenant des humates, caractérisés en ce qu'ils renferment des humates qui sont obtenus par oxydation en solution alcaline aqueuse de composés de formules générales

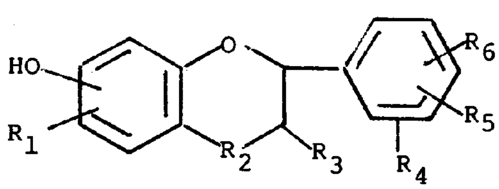 et/ou 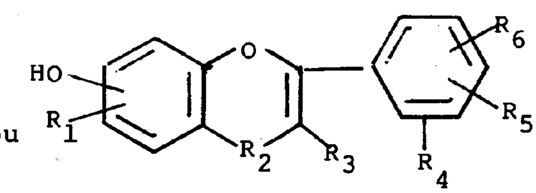

I

II

dans lesquelles $R_1$, $R_3$ et $R_4$ sont identiques ou différents et représentent un groupe OH ou l'hydrogène, $R_2$ signifie un groupe CO ou $CH_2$ et $R_5$ et $R_6$ sont identiques ou différents et représentent l'hydrogène, un groupe OH ou méthoxy, $R_4$, $R_5$ et $R_6$ n'étant pas tous simultanément l'hydrogène.

**2.** Utilisation des humates selon la revendication 1 pour la préparation d'un agent contre le rétrovirus.

**3.** Utilisation des humates selon la revendication 1 pour la préparation d'un agent anti-HIV.